# EUROPEAN PATENT APPLICATION

(11) **EP 2 684 578 A1**
(43) Date of publication of application: **15.01.2014**
(21) Application number: 13156600.2
(22) Date of filing: 25.02.2013
(51) Int. Cl.: A61M 16/06, A62B 17/04

(54) **Helmet for non-invasive ventilation of patients**

(30) Priority: 13.03.2012 IT RE20120018
(71) Applicant: Intersurgical S.P.A., 41037 Mirandola, Modena (IT)
(72) Inventor: Rossi, Paolo, 41037 Mirandola, Modena (IT)
(74) Representative: Corradini, Corrado

(57) **Abstract**

A helmet (10) for non-invasive ventilation of patients comprising:
- a container body (20) in which there can be housed the head of a patient, provided with at least one optically transparent portion and with an open end to which there is associated at least one rigid annular body (21);
- an elastically yieldable collar (30) which can be sealingly coupled to the neck of the patient and associated to the rigid annular body,

whose distinguishing characteristic lies in the fact that the collar (30) comprises at least one connection fitting (36) adapted to connect the external environment and the internal environment to the helmet.

## Description

### FIELD OF THE INVENTION

The present invention regards a helmet for non-invasive ventilation of patients.

More in particular, the invention regards a helmet for the artificial breathing of patients without using masks or tracheal tubes.

### PRIOR ART

As known, generally used for non-invasive ventilation of patients are helmets which are generally made up of a substantially cylindrical container body to which there are connected air and oxygen inlet and outlet ports and which is provided with a collar made up of a thin membrane made of elastically yieldable plastic material coupled to the neck of the patient to provide sealing.

Such collar is made up of an extremely flexible film which adheres to the skin of the patient without exerting pressure.

Such helmets are used in oxygen therapy and for ventilating patients with continuous positive pressure, the so-called CPAP and NIV.

However, the helmets for non-invasive ventilation of the known type reveal some drawbacks related to the fact that removal thereof from the head of the patients often requires subjecting the latter to complex and uncomfortable operations, disturbing the patients in question especially if the health conditions thereof are highly critical.

Such drawback is accentuated by the fact that the helmet should be well fitted in the head of the patient and subsequently removed therefrom, not only at the beginning and at the end of the therapy, but also every time there arises the need to gain access to the head of the patient for cleaning operations, routine interventions or extraordinary interventions, for example, in case of emergency.

It can be observed that, especially in case of emergency intervention, the operations of removing the helmet should be extremely prompt.

Furthermore, as known, when using the helmet the head of the patient can be connected to cables, probes or catheters which must reach - from outside - the head (mouth or nose) of the patient arranged in the container body. This is normally attained by providing connection fittings, also referred to as fairleads, provided on the container body or on a rigid connection ring between the container body and the flexible collar.

Upon connection to the patient, these probes, cables or catheters should be handled with care and must be in a stable position so as not to jeopardize the patient's health.

In order to overcome the aforementioned drawbacks and facilitate the operations of applying cables, probes or catheters to the head of the patient, there is known the use of helmets for non-invasive ventilation of patients whose container bodies are provided, at the front part thereof, with access openings, which are usually hermetically closed by closure elements, such as for example zips or rigid unions also referred to as portholes.

Should there arise the need for direct intervention to the face of the patient, the personnel may open the closure elements and gain free access to the face of the patient by means of the access opening.

However such helmets reveal some drawbacks due to the fact that both the zip and the union/porthole are not always efficient besides the fact that the opening thereof is not always easy and quick to perform for the designated personnel.

Furthermore, in order to have comfortable access to the face of the patient the access opening should be necessarily sufficiently wide at least to allow passing the hands of the designated operator to perform the intervention operations or such to allow uncovering the portion of the container body above the opening so as to free the head of the patient.

The required width of the access opening, alongside the fact that the container body is often made of a substantially soft material, amplifies the complexity of the action when opening the closure elements, thus limiting the range of use of such helmets.

Document US 5,819,728 illustrates an example of a helmet of the described type, which is essentially used in oxygen-based therapies used in a hyperbaric chamber.

The helmet described in the aforementioned document comprises a container body in which there can be housed the head of the patient and having a first rigid ring fixed at an open end thereof.

The helmet also comprises a second rigid ring provided with a flexible collar adapted to fasten against neck of the patient.

The first and the second rigid ring are sealingly coupled to each other by means of an annular gasket, of the O-ring type, which is interposed between the outer side of one of the two rings and the inner side of the other ring. The lateral sides of the gasket intervene both for providing sealing against gas leakage from the helmet for securing the two rigid rings together.

Such helmets of the known type, which are exclusively used for hyperbaric oxygen therapy, in which there is present a positive pressure within the hyperbaric chamber, cannot be used for non-invasive ventilation therapy, CPAP, in that the internal positive pressure that the helmet is subjected to, would overcome the retention force of the rings, radially exerted by the gasket, thus opening the helmet.

Even were the pressure of the gases within the helmet unable to overcome the retention force that the gas exerts on the rigid rings, the radial gasket would not guarantee the hermetic sealing of the helmet, where subjected to a positive and high internal pressure.

In these types of helmets there are no fairleads, but the only connectors present, which are the air inlet connector and the exhausted gas discharge connector, are provided on the second rigid ring.

The arrangement of such through holes on the rigid ring however reveals drawbacks, including the fact that they represent critical points for the sealing connection of the components the helmet is made of.

Moreover, a breathing apparatus is shown in the patent application nr. US 2007/0084463, the breathing apparatus comprising a hood, which surrounds the user's head and contain breathable atmosphere, and a housing suitable for providing air purification and oxygen enrichment for the atmosphere within the hood.

Such a breathing apparatus is designed to provide protection from dangerous external environments, such as smoke of fire, or chemical, biological, radiological or nuclear hazards.

The hood includes a neck seal which functions like an elastomeric membrane allowing the opening to expand to allow the user's head into the hood.

The housing is connected to the internal volume of the hood via two hoses placed on the neck seal.

The hood is free of rigid structural elements such as to be folded into a small pouch.

Moreover, in these types of hoods there are no fairleads, but the only connectors present, which are the air inlet hose and the exhausted gas discharge hose, are provided on the neck collar.

An object of the present invention is to overcome the previously mentioned drawbacks of the prior art, through a simple, rational and inexpensive solution.

Such objects are attained by the characteristics of the invention indicated in the independent claim. The dependent claims outline preferred and/or particularly advantageous aspects of the invention.

### DESCRIPTION OF THE INVENTION

In particular, the invention provides a helmet for non-invasive ventilation of patients comprising:
- a container body in which there can be housed the head of a patient, provided with at least one optically transparent portion and with an open end to which there is associated at least one body shaped to form a rigid ring; and
- an elastically yieldable collar which can be sealingly coupled to the neck of the patient and associated to the rigid annular body and
- an inlet pipe for introducing air into the container body and an air outlet pipe, for example associated to at least one between the container body and the rigid annular body.

According to the invention, the collar comprises at least one connection fitting, of the fairleads type, adapted to connect the external environment and the internal environment to the helmet.

Due to this solution, the cables, probes or catheters are connected to the patient in a safe and fixed manner; in addition, the hermetic sealing of the helmet is guaranteed under any configuration of the helmet.

In particular, if the helmet is of the openable type the fairleads provided in the collar facilitate the fixing of the cables, probes or catheters to the face of the patient when it is open, moreover the cables, probes or catheters remains fixed respect to the hood portion which can be removed from the patient's head, and simultaneously it does not interfere with the connection between the rigid rings when the helmet is closed on the patient's head.

Furthermore, if the helmet is of the closed type the fairleads provided in the collar facilitate the fixing of the cables, probes or catheters to the face of the patient through the near opening of the collar elastically expandable. BRIEF

### DESCRIPTION OF THE DRAWINGS

Further characteristics and advantages of the invention will be apparent from reading the following description provided by way of non-limiting example, with reference to the figures illustrated in the attached drawings.
Figure 1 is an exploded axonometric view of a first embodiment of a helmet according to the invention.
Figure 2 is a front exploded view of the helmet of figure 1.
Figure 3 is a side view of the helmet of figure 2 from the left in an assembled configuration.
Figure 4 is a top view of the helmet of figure 3.
Figure 5 is the view along the line of section V-V of figure 3.
Figure 6 is an enlargement of the detail VI of figure 5.
Figure 7 is an exploded axonometric view of a second embodiment of a helmet according to the invention, partly worn by a patient.

### PREFERRED EMBODIMENT OF THE INVENTION

With particular reference to such figures, a helmet for non-invasive ventilation of patients in general is indicated in its entirety with 10.

The helmet 10 comprises a container body 20 which is advantageously made up of a cylindrical element, closed at one end and open at the opposite end, made of optically transparent non-dilatable though flexible material.

The container body 20 at the lower open end thereof is advantageously connected to a first rigid ring 21, through thermo-sealing or any other fixing technique capable of guaranteeing hermetic and stable sealing between the two.

The container body 20 is provided with an inlet pipe 22 for introducing air, an air outlet pipe 23, as well as possible closable openings for access thereinto and so on and so forth and with an anti-suffocation valve 24, i.e. a two-directional valve that is capable of placing the external of the container body 20 in communication with the internal thereof.

Some embodiments of the helmet may provide for that the container body 20 be provided with openings that can be closed by means of a zip (or any other means) to provide a further access to the patient.

The helmet 10 also comprises a collar 30 which is, advantageously, made of elastically yieldable material to be sealingly coupled to the neck of the patient, which is connected to a second rigid ring 31, for example by means of fixing techniques capable of guaranteeing hermetic sealing between the collar 30 and the second rigid ring 31.

The second rigid ring 31, which for example is coated with soft material, can be sealingly removably associated to the first rigid ring 21 (also for example coated with soft material), as more apparent in the description that follows.

It cannot be excluded that the inlet pipe 22 and the outlet pipe 23 will be provided on the rigid ring (i.e. on the only rigid ring, in case of helmets of the closed type, or on the second rigid ring 31, in case of helmets of the openable type)

The helmet 10 comprises bayonet-like coupling means adapted to removably couple - with respect to each other - the first and the second rigid ring, respectively 21 and 31.

The bayonet-like coupling means comprise two coupling elements 25 and 32 which are associated, respectively, to the first rigid ring 21 and to the second rigid ring 31.

The coupling elements, in particular, comprise a plurality of pins 32, for example fixed to a lateral surface of the first rigid ring 21, and a respective plurality of housing seats 25, for example obtained in the second rigid ring 31.

In practice, each pin 32 is adapted to be removably inserted into a housing seat 25 following a slight mutual rotation between the first and the second rigid ring with respect to the mutual approaching axis.

In the embodiments shown in the figures, the second rigid ring 31 is adapted to be substantially fittingly inserted into the first rigid ring 21, coaxially; the bayonet-like coupling means comprise a plurality of pins 32, which project radially from the external lateral surface of the second rigid ring 31 and they are substantially equally spaced from each other, and a corresponding plurality of housing seats 25, obtained in the first rigid ring 21 and also equally spaced from each other.

Each housing seat 25 is substantially configured to form an extended slot (substantially L-shaped) which defines a first open section 251, at the lower edge of the first rigid ring 21, adapted to allow the access of the pin 32.

The first section 251 has an inclination with main direction parallel to the mutual approaching axis (vertical in the figure).

Each housing seat also comprises a second section 252, consecutive to the first, which is inclined with main tangential direction (horizontal in the figure). The second section 252 has a width transverse to the sliding direction of the pin 32 substantially equivalent to the width of the pin 32, so that the latter can slide substantially at contact with the walls of the second section 252.

Each second section defines an engagement portion 253 (the lower wall of the second section or both walls of the second section), for example concave (with concavity facing towards the internal of the container body 20) or rectilinear, in which there can be stably housed one of the pins 32 when the helmet 10 is in configured assembly, i.e. when the two rigid rings 21 and 31 are stably coupled to each other.

At least two pins 33 of the plurality of pins 32 - which are arranged substantially diametrically opposite with respect to each other and are adapted to be positioned laterally on the patient, once the helmet 10 is worn by the patient - have a free end configured to be gripped, having for example an ergonomic shape.

Pegs 26, also adapted to be gripped, are provided on the sides of the housing seat 25 which houses such pins 33 which can be gripped. The mutual rotation of the two rigid rings 21 and 31 occurs by mutually approaching or moving the pins 33 and the pegs 26 apart (in one or the other direction of rotation) obtained using only two fingers of the hands of the operator.

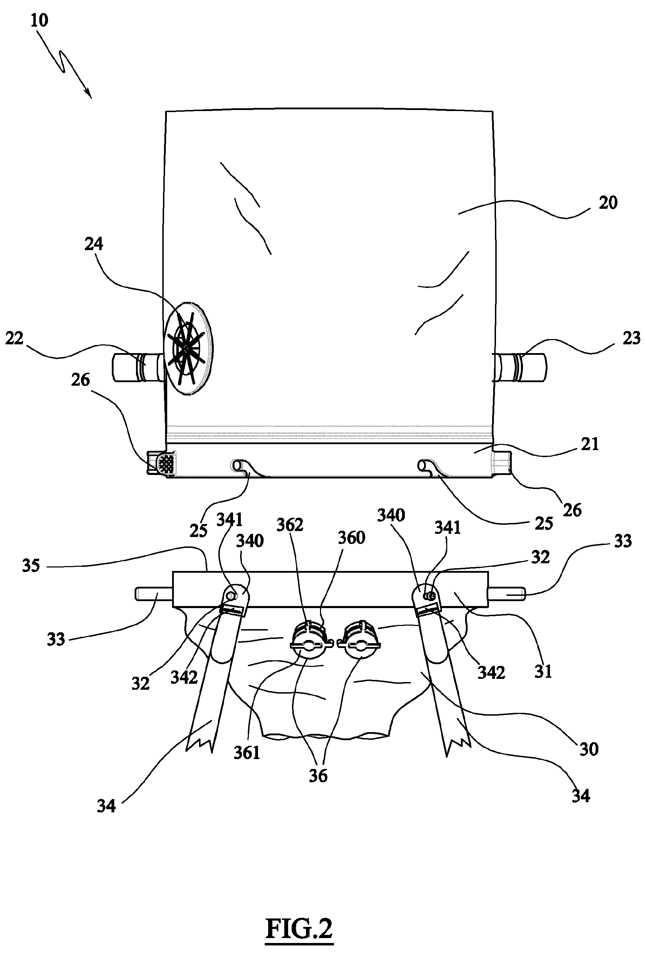

For the sake of exhaustive description it should be observed that the helmet 10 is provided with a pair of harnesses 34 (figure 2) which are of the armpit type and which are engaged with some of the pins 32 (not the pins 33 and adapted to be arranged at a front and rear position once the patient wears the helmet) and which, advantageously, are coated with soft anti-decubital material with antibacterial treatment; such harnesses have the function of preventing the helmet 10 from raising due to the positive pressure therein.

In the preferred embodiment shown in figure 2 on four of the pins 32 there are respectively inserted four lugs 340 (of which the figures show only the two front ones) provided with a hole 341 which is fittingly inserted on the pin 32 remaining axially fixed therein, so that each lug 340 is rotatably associated to the second rigid ring 31 with respect to the radial axis of the pin 32 to which it is fixed.

Each lug 340 also comprises an extended slot 342 in which there is slidably inserted the end of the harness 34 so as to be fixed therein, for example by bending the end provided with fixing means of the folded strip, for example of the Velcro type or any other suitable means.

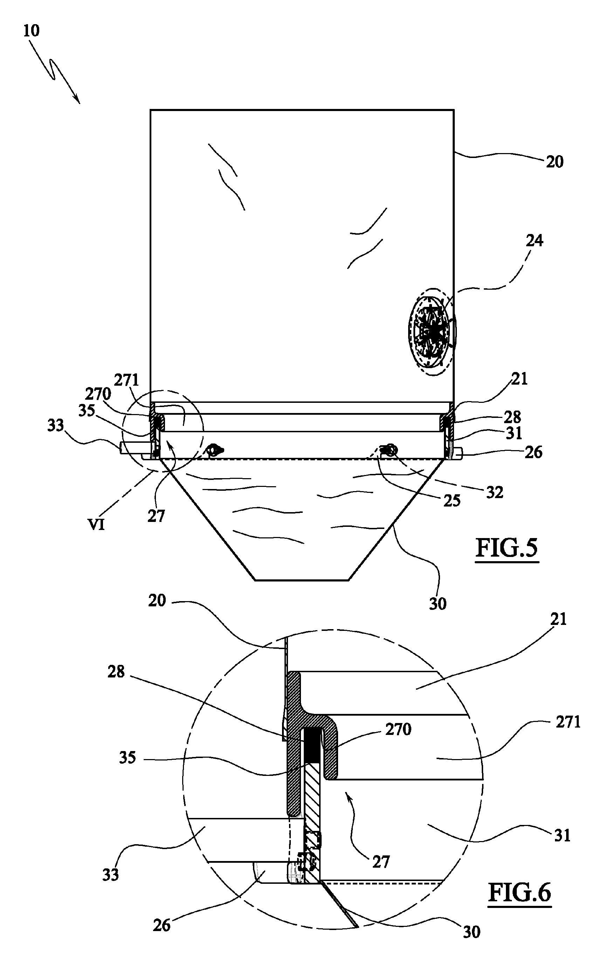

One between the first and the second rigid ring, respectively 21 and 31, comprises an annular seat 27 adapted to house an annular gasket 28, observable in the sectioned detail of figure 6.

The annular seat 27, in this case, has a substantially U-shaped transverse section, with concavity facing along the mutual approaching axis, and it is axially accessible.

In the represented example, the annular seat 27 is obtained at the inner lateral surface of the first rigid ring 21 and it is contoured, on one side, by the internal wall of the first rigid ring and, at the upper part and on the other side, by a strip 271 obtained in a single piece with the first rigid ring 21 and folded to form a U-shape on the rigid ring.

The annular seat 27, particularly, has a concavity facing towards the external of the container body 20 (downwards in the figure), so that it can be accessible for the insertion of the annular gasket 28 in the axial direction and from the bottom.

The annular seat 27 is obtained in proximity of the upper edge of the first rigid ring 21, while the coupling means are obtained in proximity of the lower edge of the first and the second rigid ring 21 and 31.

The annular gasket 28 is adapted to be compressed, in use, in the axial direction between the bottom 270 of the annular seat 27 and the upper edge 35 of the second rigid ring 31.

In practice the annular seat 27, is accessible in the axial direction from the bottom even by the second rigid ring 31, whose upper edge 35 is adapted to come to contact - along the entire circumferential development of the upper edge - with the exposed surface of the annular gasket 28 and press it towards the bottom 270 of the annular seat 27.

Such forced contact in the axial direction between the upper edge 35 and the annular gasket 28 allows the sealing connection between the first and the second rigid ring, respectively 21 and 31, and thus the sealing of the entire helmet 10, once worn and assembled.

The annular gasket 28 has at least one exposed surface layer (opposite to the contact one with the bottom 270 of the annular seat 27) made of a material having a low coefficient of sliding friction, for example made of EPDM rubber, closed cell polyurethane rubber or closed cell synthetic rubber or any other technically equivalent material.

In this manner, the upper edge 35 of the second rigid ring 31, which is substantially fittingly inserted into the annular seat 27 following an axial translation along the direction of mutual approach between the first and the second rigid ring 31, can slide without considerable friction on the annular gasket 28 following the low rotation between the two rigid rings 21 and 31 which allows the mutual coupling thereof.

Furthermore, the bayonet-like coupling means are configured so as to keep the annular gasket 28 compressed between the upper edge 35 and the bottom 270 of the annular seat 27 when the helmet is assembled.

In practice, the first section 251 of each housing seat 25 has a length such to allow each pin 32 an axial travel such that the upper edge 35 can slightly compress the annular gasket 28 to the axial end stop.

Furthermore, the engagement portion 253 of the second section 252 of each housing seat 25 (which, for example, can be slightly concave, parallel to the lower edge of the first rigid ring 21 or rising with respect thereto) is arranged at a distance from the lower edge of the first rigid ring 21 such to maintain a given compression of the annular gasket 28 even following the mutual rotation (for coupling) between the two rigid rings 21 and 31.

The sealing between the first and the second rigid ring, respectively 21 and 31, is further accentuated by the fact that such rigid rings are coated, as mentioned, by a soft material, for example rubber or any other material having a given resilience, such to be slightly compressed against the annular gasket 28 (in the respective areas of contact therewith) due to the action of the bayonet-like coupling means.

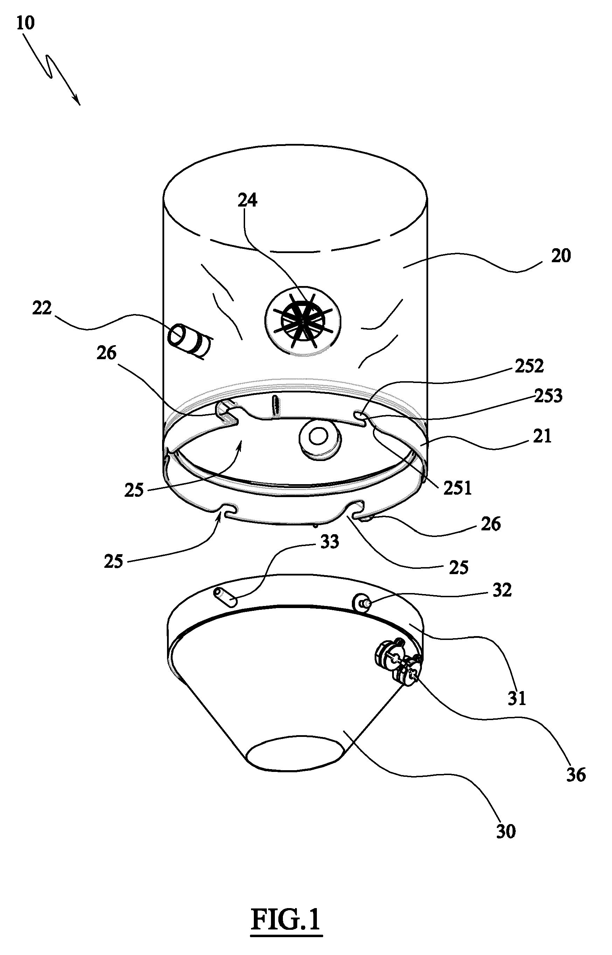

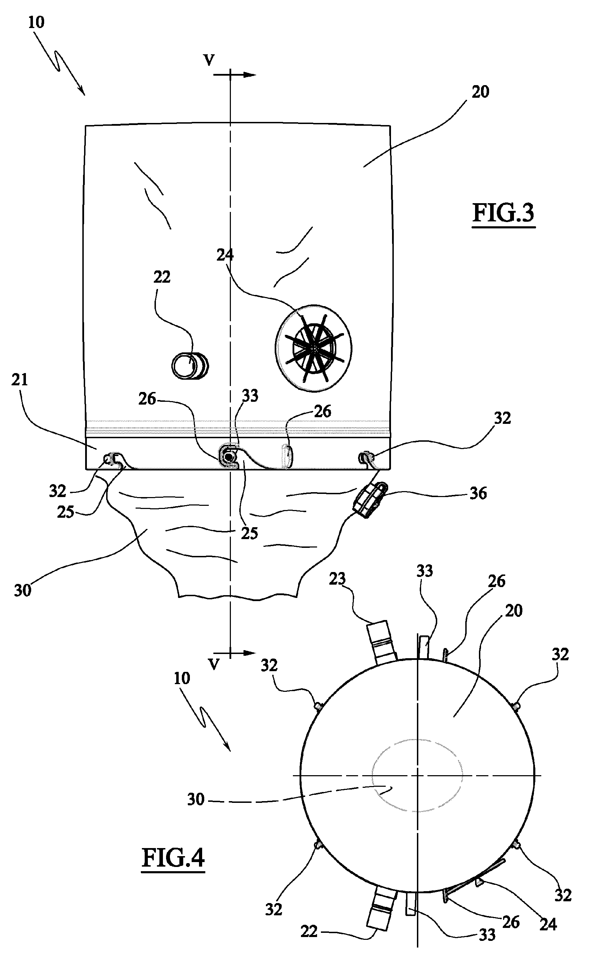

In particular, in both embodiments of the helmet 10 shown in figures 1 to 7, on the collar 30 there is associated at least one connection fitting 36 adapted to connect the external environment with the environment inside the helmet 10.

In the shown embodiments particularly, on the collar 30 there are provided through holes (for example two) to which there are associated respective connection fittings 36, for example of the fairleads type (known in the industry) or any other type of fitting for various instruments adapted for the therapy of the patient, which connect the external environment with the internal environment inside the container body 20, for example keeping the helmet 10 sealed.

Each connection fitting 36 comprises a tubular body 360, for example, welded or fixed on the collar 30.

The tubular body 360 is provided with a through cavity which for example may be closed by a piercible membrane and/or by a cover 361 fixed to the tubular body through a hinge strip 362 and adapted to sealingly close the tubular body from outside the collar 30.

In practice, due to the arrangement of the connection fittings 36 provided on the collar 30 the first rigid ring 21 and the second rigid ring 31 may not be provided with a passage between the interior and the exterior of the container body 20, as well as clefts which could jeopardize the required airtight sealing of the helmet 10, hence making the assembly of the helmet 10 easier and quicker as well as the opening thereof in emergency conditions simultaneously quick and safe and protect the patient connected to the probes/catheters.

Furthermore, the fact that the connection fittings 36 provided on the collar 30, are arranged on the part of the helmet 10 which - even when open in emergency conditions (i.e. when the container body 20 should be removed leaving the collar 30 fitted on the neck of the patient) - remains fixed with respect to the patient, allows the cables or probes fitted therein to always remain stably connected to the patient.

In the light of what has been described above, the helmet 10 operates as follows.

In order to subject the patient to non-invasive ventilation therapy it is sufficient that the patient primarily wears the collar 30, ensuring that it adheres to the neck.

In this configuration, the helmet 10 is open and the head of the patient is accessible from all parts thereof.

Furthermore, the cables, catheters or probes required to reach the mouth or nose of the patient can be easily fitted into the connection fittings 36 and be easily fixed on the face of the patient given that it is still uncovered and easily accessible.

The helmet 10 is closed by simply fitting the head of the patient into the container body 20, by approaching - through axial translation - the first rigid ring 21 to the second rigid ring 31.

Upon aligning the pins 32 of the second rigid ring 31 to the first sections 251 of the first rigid ring 21, for example by actuating - using the thumb and index finger of each hand - the pins 33 and one of the pegs 26, there follows the actuation of the two rigid rings 21 and 31 mutually approached through a slight axial translation (which partly superimposes the two rigid rings), wherein the pins 32 slide into the first section 251; at the end of the first section 251 - through the rotation of the rigid rings 21 and 31 according to a slight mutual rotation - the pins 32 may slide along the second section 252. The bayonet coupling, as mentioned, allows the annular gasket 28 to be compressed between the annular seat 27 and the upper edge 35 of the second rigid ring 31, so that the helmet 10 is substantially closed sealingly.

In such configuration, the helmet 10 is stably closed, also due to the fact that pressurized air which generates - in the helmet - a positive pressure which presses the pins 32 against the engagement portion 253 is insufflated through an inlet pipe 22.

Furthermore, such bayonet-like coupling means allow a prompt opening intervention of the helmet 10, in that by simply rotating the pins 33 and the other peg of the pair of pegs 26 using the thumb and index finger of each hand in the direction opposite to the one that caused the coupling, the pins 32 traverse the second section 252 backwards and, upon entering the first section 251, they are free to slide therealong (for example under the thrust of the positive pressure inside the helmet), so that the first rigid ring 21 slips off the second rigid ring 31 and the container body 20 can be removed from the head of the patient, leaving the collar 30 and II the instruments associated thereto in place.

The invention thus conceived can be subjected to numerous modifications and variants all falling within the inventive concept.

In practice the figures show a helmet of the openable type, in which the container body 20 can be separated from the collar 30; however it cannot be excluded that the helmet 10 can be of the conventional type, i.e. the collar 30 is fixed to the container body, for example by interposing a single rigid ring, thus obtaining a substantially monolithic body therewith.

Furthermore, all details can be replaced by other technically equivalent elements.

In practice the materials used, as well as the contingent dimensions, may vary depending on the requirements without departing from the scope of protection of the claims that follow.

## Claims

1. Helmet (10) for non-invasive ventilation of patients comprising:
- a container body (20) in which there can be housed the head of a patient, provided with at least one optically transparent portion and with an open end to which there is associated at least one rigid annular body (21,31);
- an elastically yieldable collar (30) which can be sealingly coupled to the neck of the patient and associated to the rigid annular body (21,31), and
- an inlet pipe (22) for introducing air into the container body (20) and an air outlet pipe (23),
**characterised in that** said collar (30) comprises at least one connection fitting (36), of the fairleads type, adapted to connect the external environment and the environment inside the helmet (10).

2. Helmet (10) according to claim 1, wherein said rigid annular body comprises a first rigid ring (21) fixed to the open end of the container body (20) and a second rigid ring (31) fixed to the collar (30), said first rigid ring (21) being removably sealingly associated to said second rigid ring (31) through bayonet-like coupling means.

3. Helmet (10) according to claim 2, wherein the bayonet-like coupling means comprise two coupling elements (25, 32) respectively associated to the first rigid ring (21) and to the second rigid ring (31), among which a plurality of pins (32) and respective housing seats (25) adapted to provide a mutual coupling following a slight mutual rotation between the first and the second rigid ring (21,31) with respect to the mutual approaching axis.

4. Helmet (10) according to claim 3, wherein each housing seat (25) comprises an extended slot defining a first open access section (251) inclined with main direction parallel to the mutual approaching axis and a second section (252) inclined with main tangential direction.

5. Helmet (10) according to claim 4, wherein each second section (252) defines an engagement portion (253) in which one of said pins (32) can be stably housed.

6. Helmet (10) according to claim 2, wherein at least one between the first (21) and the second (31) rigid ring comprises an annular seat (27) adapted to house an annular gasket (28), said annular seat (27) having a substantially U-shaped transverse section with concavity facing along the mutual approaching axis and being axially accessible, the annular gasket (28) being adapted to be compressed, in use, in the axial direction between the bottom (270) of the annular seat (27) and the upper edge (35) of the other between the second (31) and the first (21) rigid ring.

7. Helmet according to claim 6, wherein the second rigid ring (31) is adapted to be substantially fittingly inserted into the first rigid ring (21), the upper edge (35) of the second rigid ring (31) being adapted to be inserted into the annular seat (27) obtained at the inner surface of the first rigid ring (21), following the mutual approaching translation between the first rigid ring and the second rigid ring.

8. Helmet (10) according to claim 6 or 7, wherein the bayonet-like coupling means are configured so as to keep the gasket (28) compressed between the upper edge (35) and the bottom (270) of the annular seat (27) when the helmet (10) is assembled.
